# EUROPEAN PATENT APPLICATION

(11) **EP 3 845 553 A1**
(43) Date of publication of application: **07.07.2021**
(21) Application number: 19220117.6
(22) Date of filing: 30.12.2019
(51) Int. Cl.: C07K 14/195

(54) **CARRIER MATRIX COMPRISING DODECIN PROTEIN**

(71) Applicant: Johann Wolfgang Goethe-Universität, 60323 Frankfurt (DE); EMBL European Molecular Biology Laboratory, 69117 Heidelberg (DE)
(72) Inventor: GRININGER, Martin, 60316 Frankfurt (DE); BOURDEAUX, Florian, 60437 Frankfurt am Main (DE); GOESSNER, Ines, 60433 Frankfurt am Main (DE); REMANS, Kim, 69117 Heidelberg (DE); BESIR, Hüseyin, 69117 Heidelberg (DE)
(74) Representative: Krauss, Jan

(57) **Abstract**

The present invention relates to a carrier conjugate comprising at least one dodecin protein unit conjugated with at least one hapten and/or immunogenic and/or enzymatically active moiety. Further, the invention relates to a method for producing said conjugate and a method for producing antibodies that specifically binds to a hapten and/or immunogenic moiety of the conjugate, and to a method for performing enzymatic or diagnostic assays in vitro using said conjugate. Moreover, the invention relates to the use of said conjugate for producing antibodies that specifically bind to the epitope or epitopes contained in the moiety of said conjugate and use of said conjugate for performing enzymatic or diagnostic assays *in vitro.*

SEQUENCE LISTING

<110>  Johann Wolfgang Goethe-Universität Frankfurt
       European Molecular Biology Laboratory (EMBL)

<120>  CARRIER MATRIX COMPRISING DODECIN PROTEIN

<130>  I33490EP

<160>  18

<170>  PatentIn version 3.5

<210>  1
<211>  9
<212>  PRT
<213>  artificial

<220>
<223>  peptide linker

<400>  1

Gly Gly Gly Gly Ser Gly Gly Gly Gly
1               5


<210>  2
<211>  16
<212>  PRT
<213>  artificial

<220>
<223>  peptide linker

<400>  2

Ser Pro Ala Ala Pro Ala Pro Ala Ser Pro Ala Ser Gly Gly Ser Gly
1               5                   10                  15


<210>  3
<211>  28
<212>  PRT
<213>  artificial

<220>
<223>  peptide linker

<400>  3

Ser Pro Ala Ala Pro Ala Pro Ala Ser Pro Ala Ser Pro Ala Pro Ser
1               5                   10                  15


Ala Pro Ala Ala Ser Pro Ala Ala Gly Gly Ser Gly
            20                  25


<210>  4
<211>  12
<212>  PRT
<213>  artificial

<220>
<223>  peptide linker

<400>  4

Ser Pro Ala Ala Pro Ala Pro Ala Ser Pro Ala Ser
1               5                   10


<210>  5
<211>  16
<212>  PRT
<213>  artificial

<220>
<223>  peptide linker

<400>  5

Gly Gly Ser Gly Ser Pro Ala Ala Pro Ala Pro Ala Ser Pro Ala Ser
1               5                   10                  15


<210>  6
<211>  28
<212>  PRT
<213>  artificial

<220>
<223>  peptide linker

<400>  6

Gly Gly Ser Gly Ser Pro Ala Ala Pro Ala Pro Ala Ser Pro Ala Ser
1               5                   10                  15


Pro Ala Pro Ser Ala Pro Ala Ala Ser Pro Ala Ala
            20                  25


<210>  7
<211>  15
<212>  PRT
<213>  Homo sapiens

<400>  7

Pro Lys Gly Gly Ser Gly Ser Gly Pro Thr Ile Glu Glu Val Asp
1               5                   10                  15


<210>  8
<211>  15
<212>  PRT
<213>  Homo sapiens

<400>  8

Pro Leu Glu Gly Asp Asp Asp Thr Ser Arg Met Glu Glu Val Asp
1               5                   10                  15

<210>  9
<211>  15
<212>  PRT
<213>  Homo sapiens

<400>  9

Glu Cys Tyr Pro Asn Glu Lys Asn Ser Val Asn Met Asp Leu Asp
1               5                   10                  15


<210>  10
<211>  15
<212>  PRT
<213>  Homo sapiens

<400>  10

Val Pro Ser Asp Ser Asp Lys Lys Leu Pro Glu Met Asp Ile Asp
1               5                   10                  15


<210>  11
<211>  15
<212>  PRT
<213>  Homo sapiens

<400>  11

Asp Ser Ser Gln His Thr Lys Ser Ser Gly Glu Met Glu Val Asp
1               5                   10                  15


<210>  12
<211>  15
<212>  PRT
<213>  Homo sapiens

<400>  12

Glu Gln Ser Thr Gly Gln Lys Arg Pro Leu Lys Asn Asp Glu Leu
1               5                   10                  15


<210>  13
<211>  15
<212>  PRT
<213>  Homo sapiens

<400>  13

Ala Leu Met Val Tyr Arg Cys Ala Pro Pro Arg Ser Ser Gln Phe
1               5                   10                  15


<210>  14
<211>  15
<212>  PRT
<213>  Homo sapiens

<400>  14

Leu Val Thr Gly Glu Ser Leu Glu Gln Leu Arg Arg Gly Leu Ala
1               5                   10                  15

<210>  15
<211>  15
<212>  PRT
<213>  Homo sapiens

<400>  15

Met Lys Gly Lys Glu Glu Lys Glu Gly Gly Ala Arg Leu Gly Ala
1               5                   10                  15

<210>  16
<211>  10
<212>  PRT
<213>  Homo sapiens

<400>  16

Glu Glu Arg Arg Ile His Gln Glu Ser Glu
1               5                   10

<210>  17
<211>  10
<212>  PRT
<213>  Homo sapiens

<400>  17

Asn His Glu Gly Asp Glu Asp Asp Ser His
1               5                   10

<210>  18
<211>  7
<212>  PRT
<213>  artificial

<220>
<223>  synthetic linker

<400>  18

His His His His His His His
1               5

## Description

### FIELD OF THE INVENTION

The present invention relates to a carrier conjugate comprising at least one dodecin protein unit conjugated with at least one hapten and/or immunogenic and/or enzymatically active moiety. Further, the invention relates to a method for producing said conjugate and a method for producing antibodies that specifically binds to a hapten and/or immunogenic moiety of the conjugate, and to a method for performing enzymatic or diagnostic assays in vitro using said conjugate. Moreover, the invention relates to the use of said conjugate for producing antibodies that specifically bind to the epitope or epitopes contained in the moiety of said conjugate and use of said conjugate for performing enzymatic or diagnostic assays *in vitro.*

### DESCRIPTION

Scaffold proteins were discovered about three decades ago, and have been studied since then in their roles in cellular processes and as tools in bioengineering. In cellular processes, the role of scaffold proteins exerts from simply enhancing interaction efficiencies to altering signalling pathways. A common application of scaffolds in bioengineering is their use as carriers of haptens for immunizations and antibody production. Virus like particles (VLP) are prototypical for a scaffold in bioengineering and are used for antibody production, as vaccines or as a microreactor containing enzymes.

Alternatively, scaffolds are used in constructing enzyme hubs in which enzymes are brought into close proximity in order to enhance enzymatic pathways or to increase proteins in stability by fusion to the scaffolding solid support. Although the specific requirements for an antigen carrier may differ from the needs of an enzyme hub scaffold, both require a protein that forms or assembles into a defined structure, and then the means and methods to attach a moiety.

For being suited as carriers, proteins need to meet a set of requirements. They need to be highly water soluble, robust and stable, as well as structurally insensitive to the attached moiety. Carrier proteins should further allow the dense packing of the moiety in homovalent and ideally also in heterovalent fashion. For example, dense packing imposes advantages in immunizations. Highly repeating densely packed epitopes on particle surface facilitate B-cell activation through increased cell surface receptor oligomerization. Heterovalent coating of carriers allows antibody production against two or more targets (which can be part of a larger single target) without the need of preparing two or more separate antigens. Such strategies can enhance success rates during antibody production against a single target, if the different coating moieties originate from a larger single target, or broaden the spectrum of produced antibodies against several targets, e.g. different proteins of a pathogen. Further, heterovalent coating can be utilized to enhance the immune response by co-coupling with known or expected immunogenic moieties.

One application of protein carriers is its loading with peptides for the generation of antibodies for laboratory use and diagnostic purposes. Such antibodies are employed to identify proteins that contain the peptide sequence in complex samples, as well as to analyse them in concentration, regulation and locations in cells. For recognition of the target protein in the native/ folded state, the recognized epitope needs to be accessible by the corresponding antibody, therefore surface exposed sequence regions, like for example the protein termini, are well-suited for the peptide sequence selection. In order to enhance the chance of the production of antibodies that recognize the native/ folded state, the peptide is ideally displayed at the carrier surface in similar orientation as in in the intact target protein. In a typical peptide-carrier design for antibody production, a 10 to 20 amino acid long peptide is coupled to residues at the surface of key-hole limpet hemocyanin (KLH), bovine serum albumin (BSA) or rabbit serum albumin (RSA) via chemical-synthetic bond formation. Conjugation reactions are at risk of depending in efficiency on the type of peptide, which may lead to undefined samples in certain cases, and are limited in making heterovalent conjugates accessible. BSA, KLH and RSA bear typical carrier properties, and the conjugated peptides are exposed at the surface at high density. As the immune system recognises the entire carrier conjugate, high immunogenicity of the carrier itself is beneficial for inducing a strong immune response during immunizations. It is noted that since the entire carrier conjugate is recognized by the immune system, antibodies against the whole conjugate are produced, not only the conjugated moiety.

The dodecin protein family was recently found to be a flavin storage and buffering system that occurs in bacteria and archaea, but not in eukaryotes [1-4]. Dodecins are about 8 kDa small proteins of βαββ-topology. The monomer forms an SHS2 domain (a small curved antiparallel β-sheet that partly enwraps the helix). The assembled dodecamer of dodecins is a unique protein fold. Dodecins largely meet the requirements of protein carriers. In the native dodecameric state, dodecins are of spherical shape with 23-cubic symmetry, and the N- and C-termini are exposed at the protein surface. Dodecins show pronounced thermostability, which likely originates from extensive inter monomer β-sheet contacts and salt bridges built upon assembly of the dodecamer.

Flavins are broadly used cofactors that are involved in a variety of light-induced reactions and many redox processes, as they can catalyse the transfer of 1 or 2 electrons. Most common flavins are riboflavin, FMN and FAD, the latter two are mainly used as cofactors, while riboflavin is used as their precursor. To ensure that enough riboflavin is present for the synthesis of FMN and FAD, riboflavin-binding proteins with their function to store and transport riboflavin are utilized. Dodecins seem to fulfil a similar role.

WO 2002/032925 discloses proteins that include an immunoglobulin fold and that can be used as scaffolds for immunoglobulin. Also disclosed are nucleic acids encoding such proteins and the use of such proteins in diagnostic methods and in methods for evolving novel compound-binding species and their ligands.

It is therefore an objective of the present invention to provide dodecin as a new and improved carrier protein.

A further objective of the present invention is to provide a method for producing a conjugate comprising the dodecin and a moiety.

Another objective is the provision of a method for producing antibodies using the conjugate and the provision of a method for performing a diagnostic assay.

So far, dodecin has not been thought to have any other properties than to bind flavin and coenzyme A (CoA), but the inventors of the present invention surprisingly demonstrated the suitability of the dodecin of *Mycobacterium tuberculosis* (mtDod) as a preferred example for a new scaffold protein. MtDod is a homododecameric protein of spherical shape, high stability and of robust assembly.

The inventors surprisingly discovered that dodecins are suitable as carriers for other units/moieties and have a number of advantages. Dodecin has a unique folding and is not significantly sequence-similar to eukaryotic proteins or to other proteins in *E. coli.* Thus, antibodies produced by dodecin antigen fusions should not show false signals or a high signal background ("matrix effects"). Dodecin as a carrier matrix thus complements existing carrier proteins and can easily be integrated into existing methods. Compared to other carrier proteins, dodecin is very stable, can be purified with simple protocols and can be stored over a longer period of time without any problems. Dodecin is therefore an advantageous alternative matrix to KLH, BSA, ovalbumin and MAP.

Dodecin is a versatile matrix for flexible or rigid peptides and small proteins. In addition to its high stability, dodecin can be produced with high yields in *E. coli,* as shown by the purification of up to several 100 mg dodecin-peptide fusions per litre of culture. Proteins fused with dodecin are presented on the dodecin surface and remain demonstrably accessible and functional.

The high stability, easy production and high yields also make mtDod and other dodecins interesting for biotechnological applications where defined particles are required, e.g. diffusion measurements, and in combination with docking domains non-covalently or covalently (e.g. SpyCatcher System) linking molecules for the formation of biomaterials and enzyme scaffolds. The formation of heterododecamers (in vivo or in vitro) also offers possibilities for pull-down assays.

In a first aspect, the invention relates to a conjugate comprising at least one dodecin protein unit conjugated with at least one of hapten and/or immunogenic and/or enzymatically active moiety. Either said at least moiety is directly fused at the gene level, or dodecin is posttranslationally complexed/conjugated with said least one moiety.

In a second aspect, the invention relates to a composition comprising the conjugate as described herein and at least one of a suitable carrier, excipient, enzyme substrate and/or adjuvant.

In a third aspect, the invention relates to a nucleic acid encoding for a conjugate as described herein.

In a fourth aspect, the invention relates to a vector comprising the nucleic acid as described herein, in particular an expression vector expressing or overexpressing said nucleic acid.

In a fifth aspect, the invention relates to a recombinant host cell comprising the nucleic acid, or the vector as described herein.

In a sixth aspect, the invention relates to a method for producing a conjugate as described herein, wherein the method comprises the following steps of suitably culturing a recombinant host cell comprising and expressing the nucleic acid, or the vector as described herein, and isolating the conjugate from the cell and/or the culture medium thereof.

In a seventh aspect, the invention relates to a method for producing a conjugate as described herein, wherein the method comprises the following steps of suitably culturing a recombinant host cell comprising and expressing a nucleic acid, or a vector encoding a dodecin protein unit, isolating the dodecin; and coupling a moiety that contains a desired epitope (likely a hapten), is chemically or enzymatically modifiable or is enzymatically active via a functional group to the isolated dodecin.

In an eighth aspect, the invention relates to a method for producing an antibody that specifically binds to the moiety of the conjugate as described herein, comprises the steps of suitably immunizing a subject with the conjugate, and isolating an antibody specifically binding to the desired epitope containing moiety of said conjugate from the subject.

In a ninth aspect, the invention relates to a method for performing a diagnostic assay in vitro, wherein the method comprises the following steps of: immobilizing the conjugate as described herein on a solid carrier; adding of an enzyme substrate; determining the amount of converted enzyme substrate.

In a tenth aspect, the invention relates to a use of the conjugate as described herein, for producing a specific antibody that specifically binds to the hapten of the conjugate.

In an eleventh aspect, the invention relates to a use of the conjugate as described herein, for performing a diagnostic assay *in vitro.*

In the first aspect, the invention relates to a conjugate comprising at least one dodecin protein unit conjugated with at least one hapten and/or at least one immunogenic and/or at least one enzymatically active moiety.

The term "conjugate" as used herein shall also include any additional compound/composite consisting of more than this at least one dodecin protein unit and this at least one hapten and/or immunogenic and/or enzymatically active moiety.

In the context of the present invention the term "protein" is used to denote a polymer composed of amino acids joined by peptide bonds. It refers to a molecular chain of amino acids, and does not refer to a specific length of the product and if required can be modified *in vivo* or *in vitro,* for example by glycosylation, amidation, carboxylation or phosphorylation. Amino acid chains with a length of less than approx. 100 amino acids are called "peptides". The terms "peptides", and "proteins" are included within the definition of "polypeptides". A "peptide bond" is a covalent bond between two amino acids in which the α-amino group of one amino acid is bonded to the α-carboxyl group of the other amino acid. All amino acid or polypeptide sequences, unless otherwise designated, are written from the amino terminus (N-terminus) to the carboxy terminus (C-terminus).

In the context of the present invention the term "dodecin" is used to denote both a single protein unit as well as the up to dodecameric arrangement of the protein (sub)units. Thus, included are 1, 2, 3, 4, 5, 6, 7, 8, 9, 10, 11, and 12 arrangement of the protein (sub)units. In some embodiments, the term includes mixtures of dodecin units derived from different organisms, provided that they can form dimers or multimers as above. Also, mixtures of fusion and non-fusion or complexed and non-complexed dodecin protein units can be used in an arrangement, such as the dodecameric arrangement, as long as at least one conjugated unit is included.

As used herein the term "immunogenic" means the ability of a compound to trigger an immune response of the immune system in the body of a subject. To possess this property, the immunogenic compound must be recognized by the subject's body as a foreign substance. Factors involved are molecular size (objects smaller than 5000 Da are not likely to be recognized), overall structure (like tertiary structure) and composition (like amino acid sequence). Immunogenic active substances can be, for example, cells, cell extracts, pathogens, viruses, proteins, peptides, carbohydrates, or lipids, or parts thereof, but without being limited to them.

The term "enzymatically active" as used herein means a moiety in which the function as a biocatalyst for accelerating chemical reactions is inherent. The enzymatically active moieties are mostly proteins. The "enzymatically active" moieties include entire proteins or peptides, or parts thereof, as long as the enzymatic activities are maintained. One example is horseradish peroxidase. The enzyme catalyses the reduction of various peroxides, mostly hydrogen peroxide. For example one possible substrate is luminol or other dioxetanes that can be catalysed by this peroxidase. In addition, horseradish peroxidase forms coloured or fluorescent reaction products with various chromogens.

In an embodiment of the herein disclosed invention, said at least one hapten and/or immunogenic and/or enzymatically active moiety is complexed with and/or genetically fused to said dodecin protein unit.

The term "complexed" as used herein means that at least one dodecin protein unit and at least one hapten and/or immunogenic and/or enzymatically active moiety are bound to each either via a covalent bond and/or a non-covalent interaction, such as ion bonds, hydrogen bonds, Van der Waals force and hydrophobic interactions. Furthermore, the above units can be complexed through linkers and binding groups, e.g. biotin/streptavidin, and the like. The complexation can be carried out posttranslationally via chemical reactions. The term "genetically fused" shall mean that the nucleic acids encoding for the individual components of the conjugate are already fused at the genetic level and are expressed together as fusion proteins. The "genetic fusion" is based on an artificial nucleic acid consisting of the nucleic acid encoding for the dodecin protein unit and the nucleic acid encoding for the hapten and/or immunogenic and/or enzymatically active unit which is the genetic template for protein translation. Hence, a "fusion protein" relates to an artificial proteinaceous construct and means a protein comprising at least two different amino acid sequences which are defined by their origin and/or by special functions.

In a further embodiment of the present invention, said dodecin protein is a dodecamer of twelve units of said dodecin protein, wherein said dodecamer comprises at least one conjugated dodecin protein unit conjugated with said at least one hapten and/or immunogenic and/or said enzymatically active moiety.

As used herein, the term "dodecamer" describes the quaternary structure of the protein complex consisting of 12 dodecin units. The examination of the crystal structure of the dodecin dodecamer showed that the three-stranded antiparallel beta sheets of the subunits line the interior of the dodecin dodecamer, while the alpha helices face the outside of the dodecamer. The dodecin subunits are strongly involved in the contacts within the dodecamer, as 42% of the monomer surface is buried in the contacts of the subunit. In contrast to other known flavoproteins, which only bind flavin monomers, the structure of the dodecin comprises six flavin dimers. [1]

In another further embodiment of the herein described invention, both termini of the amino acid chain of said dodecin protein unit are located on the outer surface of said dodecamer.

The term "termini" as used herein means the C-terminus and the N-terminus. The C-terminus or C-terminal region of a protein or peptide is the part of the molecule that contains the free carboxyl group (-COOH) connected to the Cα atom not involved in a peptide bond. The molecule end of a peptide opposite the C-terminus is called the analogue N-terminus. The N-terminus or N-terminal region of a protein or peptide is the part of the molecule that contains the free amino group (-NH2) connected to the Cα atom not involved in a peptide bond. Since a carboxy group in a polypeptide is always linked to an amino group of the following amino acid at α, an amino group remains at the beginning of a polypeptide or protein and a carboxy group remains free at the end. In the form of the dodecamer, both C-terminus and N-terminus of a dodecin unit are exposed on the outer surface of the complex and allow the dodecin dodecamer to be charged with 1 to 24 moieties on its surface.

In yet another embodiment of the present invention, said moiety is selected from an antigen, an enzyme, a protein, a lipid, and a small molecule.

The term "moiety" as used herein, refers to a specific segment or functional group selected from an antigen, an enzyme, a protein, a lipid, and a small molecule, wherein all functional elements necessary for the performance or the respective functions of an antigen, an enzyme, a protein, a lipid, and a small molecule are maintained.

As used herein, the term "antigen" refers to a substance capable of eliciting an immune response. Antigens are usually proteins, peptides, carbohydrates or other complex molecules known by the skilled person. As used herein, the term "enzyme" means a biochemical catalyst that supports the conversion of a substrate specific to it but is not itself chemically modified. Most enzymes belong to the group of proteins, with the exception of ribozymes, which are made up of RNA. The term "lipid" as used herein refers to the totality of fats and fat-like substances. Lipids are chemically heterogeneous substances that dissolve poorly in water, but well in nonpolar solvents. The term "small molecules" as used herein comprises low molecular weight compounds with a low molecular mass. This refers to a class of active substances with a molecular mass not exceeding approximately 800 g-mol-1.

As used herein, the term "hapten" refers to a substance capable of eliciting an immune response (e.g. being an antigen as above), in particular a substantial immune response, like the generation or binding of an antibody, only in the context/form of the conjugate as disclosed herein. Examples are peptides that cause no or only a low immune response, but become immunogenic after being conjugated according to the invention.

In another embodiment of the present invention, said conjugation is covalently or non-covalently via a functional group. The term "covalent" as used herein refers to an atomic bond formed by electrostatic attraction between two atoms. The term "non-covalent" refers to chemical interactions between atoms in which they do not share electron pairs. A distinction is made between non-covalent bonds in hydrogen bonds, Van-der-Waals interactions, hydrophobic interactions and electrostatic interactions.

In yet another embodiment of the present invention, said functional group is selected from a thiol group, an amino group, a carboxy group and an azide group.

In one embodiment of the present invention, said fusion is direct (on the gene-level) or indirect (post-translationally via docking domains) via a suitable linker group or sequence.

The term "suitable linker" as used herein describes a group or sequence that allows the moiety as described above and the dodecin protein unit to be linked so that both moiety and dodecin protein unit are linked together without for example aggregating. After the linkage, the linker should be robust and should show no further reactivity, therefore only serve to connect the single components. The linker according to the present invention can be flexible or rigid. Suitable linkers are known to the skilled person.

In a further embodiment of the present invention, said dodecin protein unit conjugated with at least one hapten and/or immunogenic and/or enzymatically active moiety is suitably labelled. In the context of the present invention, the term "suitably labelled" means that the conjugate may contain additional markers, such as non-protein molecules such as nucleic acids, sugars, or markers for radioactive or fluorescent labelling. For example, these labels can be used to determine or verify the fusion of the dodecin protein unit with the hapten and/or immunogenic and/or enzymatically active moiety.

In a further embodiment of the present invention, said dodecin protein unit is derived from a bacterium or archaea selected *from Halobacterium salinarum, Halobacterium halobium, Streptomyces davaonensis, Streptomyces coelicolor, Chlorobium tepedium, Sinorhizobium meliloti, Bordetella pertussis, Bordetella bronchiseptica, Pseudomonas aeruginosa, Pseudomonas putida, Acinetobacter baumannii, Geobacter sulfurreducens, Thermus thermophilus,* and preferably from *Mycobacterium tuberculosis.*

In the second aspect, the invention relates to a composition comprising the conjugate as described herein before, and at least one of a suitable carrier, excipient, enzyme substrate and/or adjuvant.

The term "composition" as used herein refers to a formulation, which is present in such a form that the conjugate and at least one of a suitable carrier, excipient, enzyme substrate and/or adjuvant contained therein are effective. A composition of the present invention may be produced by a variety of methods known in the art. It may be necessary to treat or modify the components comprised by the composition with a material to prevent their inactivation. For example, the composition may be prepared in a suitable buffer to prevent denaturation of the protein units while maintaining the activity of the hapten and/or immunogenic and/or enzymatically active moiety.

A "suitable carrier" or "excipient" refers to ingredients in a formulation, other than an active ingredient, which are nontoxic to a subject. Suitable carriers include any and all solvents, dispersion media, coatings, antibacterial and antifungal agents, isotonic and absorption delaying agents, and the like that are compatible. The prevention of the presence of microorganisms can be ensured both by sterilization procedures, and by the use of various antibacterial and antifungal agents.

An "enzyme substrate" is a substance that is converted in an enzymatically-controlled reaction. According to the present invention, it is a substrate that is converted by the enzymatically active moiety. There is a variety of known substrates in the prior art, the selection of which depends on the respective enzymatically active moiety.

An "adjuvant" as used in the context of the present invention, is an agent that enhances the overall immune response. Common adjuvants include suspensions of minerals (alum, aluminium hydroxide, aluminium phosphate); emulsions, including water-in-oil, and oil-in-water (and variants thereof, including double emulsions and reversible emulsions), liposaccharides, lipopolysaccharides, immunostimulatory nucleic acids (such as CpG oligonucleotides), liposomes, Toll-like Receptor agonists, and various combinations of such components.

In the third aspect, the invention relates to a nucleic acid encoding for a conjugate as described herein before.

The term "nucleic acid" used in the present invention encodes a dodecin protein unit and a moiety, as described above, which may be genetically fused. The nucleic acid of the present invention may be, for example, DNA, cDNA, RNA or synthetically produced DNA or RNA or a recombinantly produced chimeric nucleic acid molecule comprising one of these nucleic acids alone or in combination.

In the fourth aspect, the invention pertains to a vector comprising the nucleic acid as described above, in particular an expression vector expressing or overexpressing said nucleic acid.

The term "vector" may comprise further genes such as marker genes, which allow for the selection of the vector in a suitable host cell and under suitable conditions. Expression of said nucleic acid or vector comprises transcription of the nucleic acid into a translatable mRNA. Usually a vector comprises regulatory sequences ensuring initiation of transcription. Beside elements which are responsible for the initiation of transcription such regulatory elements may also comprise transcription termination signals downstream of the nucleic acid.

In the fifth aspect, the invention relates to a recombinant host cell comprising the nucleic acid, or the vector as described above. The term "host cell" as used herein means a prokaryotic or eukaryotic cell. The nucleic acid or vector of the present invention, which is present in the host cell, may either be integrated into the genome of the host cell or it may be maintained extra-chromosomally. For example, the host cell can be a bacterial, insect, fungal, plant, animal or human cell. The term "prokaryotic" is meant to include all bacteria which can be transformed or transfected with a DNA or RNA molecules for the expression of a moiety genetically fused to a dodecin protein unit as described herein. The term "eukaryotic" is meant to include yeast, higher plant, insect and mammalian cells. Once the nucleic acid or vector has been incorporated into the appropriate "host cell", the host cell is maintained under conditions suitable for high level expression of the nucleic acid or vector.

In the sixth aspect, the invention relates to a method for producing a conjugate as described herein above, wherein the method comprises the following steps of suitably culturing a recombinant host cell comprising and expressing the nucleic acid, or the vector as described above, and isolating the conjugate from the cell and/or the culture medium thereof.

The transformed host cells can be grown in fermenters and cultured according to techniques known in the art to achieve optimal cell growth. Once expressed, the conjugate can be purified according to standard procedures of the art, including ammonium sulphate precipitation, affinity columns, column chromatography, such as size exclusion chromatography (SEC), gel electrophoresis and the like. The conjugate of the invention can then be isolated from the growth medium, cellular lysates, or cellular membrane fractions. The isolation and purification of the conjugate may be by any conventional means such as, for example, preparative chromatographic separations.

The terms "isolating" or "isolation" when referring to a molecule, for example, a conjugate according to the present invention, is a molecule that by virtue of its origin or source of derivation is not associated with naturally associated components that accompany it in its native state, is substantially free of other molecules from the same species is expressed by a cell from a different species, or does not occur in nature without human intervention. In other words, an "isolated conjugate" is one, which has been identified and separated and/or recovered from a component of its natural environment. Thus, a molecule that is chemically synthesized, or synthesized in a cellular system different from the cell from which it naturally originates, will be "isolated" from its naturally associated components. A molecule also may be rendered substantially free of naturally associated components by isolation, using purification techniques well known in the art. Molecule purity or homogeneity may be assayed by a number of means well known in the art. For example, the purity of a polypeptide sample may be assayed using polyacrylamide gel electrophoresis and staining of the gel to visualize the polypeptide using techniques well known in the art. For certain purposes, higher resolution may be provided by using HPLC or other means well known in the art for purification.

In the seventh aspect, the invention relates to a method for producing a conjugate as described above, wherein the method comprises the following steps of suitably culturing a recombinant host cell comprising and expressing a nucleic acid, or a vector encoding a dodecin protein unit, isolation said dodecin; and coupling an hapten and/or immunogenic and/or enzymatically active moiety via a functional group to said isolated dodecin.

Such coupling occurs chemically after expression and if required isolation of the dodecin protein unit at the N-terminus, C-terminus, fused moieties at the termini or any other position of the dodecin protein and dodecin-conjugate units. The appropriate coupling methods to be applied are obvious to the skilled person. For example, the activation of reactive groups of the corresponding molecules by UV irradiation.

In the eighth aspect, the invention relates to a method for producing an antibody that specifically binds to the attached moiety of the conjugate as described herein above, comprises the steps of suitably immunizing a subject with the conjugate as described herein, and isolating an antibody specifically binding to the attached moiety of said conjugate from the subject.

"Antibody" and "antibodies" refer to antigen-binding proteins that arise in the context of the immune system. The term "antibody" as referred to herein, in the context of molecules binding to the attached moiety of the conjugate, includes whole, full length antibodies and any fragment or derivative thereof in which the "antigen-binding portion" or "antigen-binding region" or single chains thereof are retained, such as a binding domain of an antibody specific for the attached moiety of the conjugate (e.g. an scFv). Antibodies can be monoclonal, human, or partially or fully humanized.

In one embodiment of the method according to the present invention, the subject is a mammal. As used herein the term "subject" comprises non-human mammals, or a mammal selected from a mouse, a rat, a monkey, a rabbit, a pig, a donkey, a cow, a horse, a shark, a camelid, a sheep, or a human.

In the ninth aspect, the invention relates to a method for performing a diagnostic assay *in vitro,* wherein the method comprises the following steps of immobilizing the conjugate as described herein above on a solid carrier; adding an enzyme substrate; and determining the amount of converted enzyme substrate.

The term "solid carrier" refers to any carrier, which is insoluble and chemically inert under the reaction conditions without hindering or preventing enzymatic reactions. A large reaction area can be achieved by using very porous materials. Furthermore, the carrier must allow the substrate to flow in and out. A number of suitable carriers are known. In one embodiment of the method according to the present invention, the solid carrier is selected from glass, agarose, polymers, or metals.

In the tenth aspect, the invention relates to the use of the conjugate according to the present invention for producing an antibody that specifically binds to the moiety of the conjugate, in particular to said hapten of said conjugate.

In the eleventh aspect, the invention relates to a use of the conjugate according to the present invention for performing a diagnostic assay in vitro, for example in an enzymatic, in particular diagnostic, *in vitro* assay.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. Where an indefinite or definite article is used when referring to a singular noun, e.g. "a", "an" or "the", this includes a plural of that noun unless something else is specifically stated.

All references, patents, and publications cited herein are hereby incorporated by reference in their entirety.

### BRIEF DESCRIPTION OF THE FIGURES

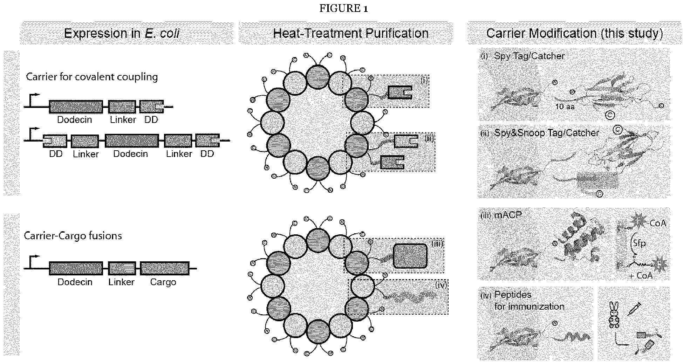

**Figure 1** shows a schematic depiction of mtDod constructs. The constructs of the present invention can, for example, be produced in *E. coli* (left) and purified by a heat treatment protocol (middle). The dodecamers preferably expose their termini at the outer surface. These termini can then be attached to/loaded with peptides, proteins and small molecules ("cargo") by attaching said cargo indirectly at "docking sites" (DD) ((i) - (iii)) or by directly fusing said cargo on the gene level (iv).

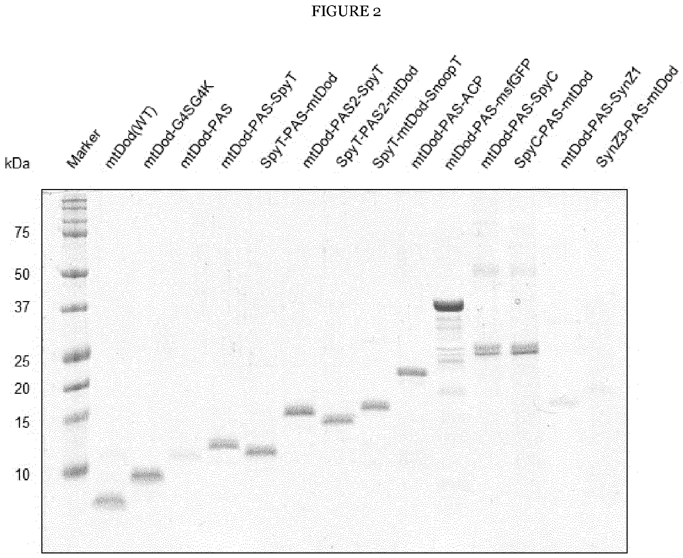

**Figure 2** shows an SDS PAGE gel of purified dodecin constructs. For full denaturing of the dodecamer an acidic loading buffer (pH 4.2) containing 3.3% SDS was used during the heat treatment (10 min, 95 °C). After the heat treatment, the pH was increased to about 6.8 using a glycerol and Tris-HCl containing buffer. For mtDod-msfGFP-H8 impurities of lower mass are observable which may be caused by mtDod with degraded msfGFP. The double bands observed for both SpyCatcher mtDod constructs seem to be caused by the acidic loading dye in combination with the SpyCatcher, as also seACP-SpyC formed double bands when the acidic loading dye was used. The origin of this behavior was not further investigated.

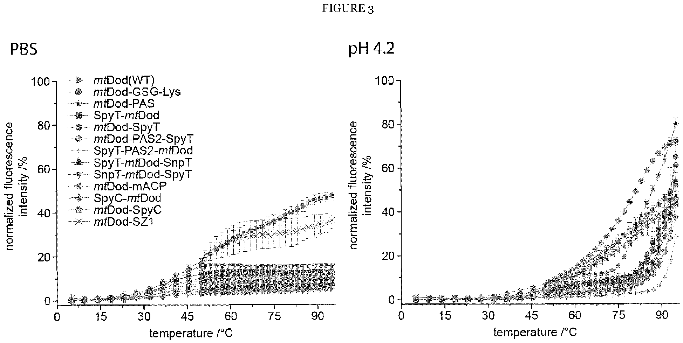

**Figure 3** shows the thermal stability of mtDod constructs determined with a termed thermocyclic fluorescence assay. The FMN fluorescence at the rebinding/cooling phase is plotted against the heating phase temperature. Increasing FMN fluorescence indicates disassembly of the dodecamer at the heating phase as the flavin cannot be rebound in the cooling phase and its fluorescence is not quenched. In PBS, no significant increase of fluorescence is observed, except for mtDod-SpyC and mtDod-SZ1, indicating that the dodecameric mtDod core structures of all other constructs does not disassemble. The minor increase of fluorescence of up to 20% around 45-50 °C, might be caused by hindered rebinding of FMN and not by disassembling of the dodecamer. At pH 4.2, all constructs show a significant increase of fluorescence indicating the disassembly of the dodecamer. Most constructs behave like mtDod(WT) and are stable to about 80 °C, except mtDod-PAS-Met, mtDod-mACP, mtDod-SpyC and SpyC-mtDod, of which the last three start denaturating already at 50 °C. mtDod-PAS-Met is only slightly less stable and starts to denature around 75 °C. Data for mtDod-SpyC and mtDod-SZ1 need careful evaluation, because both constructs have a significantly impaired FMN binding and were not saturated with FMN. The fluorescence was normalized with the maximal fluorescence measured in the heating phase corrected by the temperature-induced fluorescence decline of FMN.

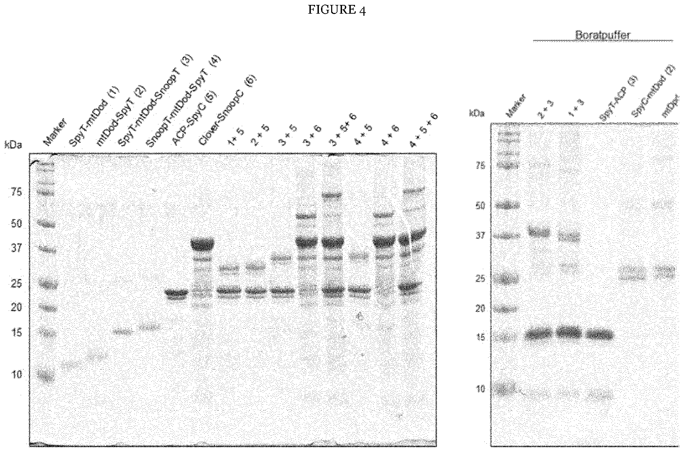

**Figure 4** shows a SDS PAGE of the SpyTag/-Catcher and SnoopTag/-Catcher reactions. Left: Reactions of mtDod Spy-/SnoopTag constructs with seACP-SpyCatcher and/or mClover3-SnoopCatcher. Right: Inverse reactions of mtDod SpyCatcher constructs with SpyT-seACP. In all reactions, bands of higher mass representing the reaction product are observed. For both mtDod-SpyCatcher constructs and seACP-SpyCatcher double bands are observed, caused by the acidic loading dye.

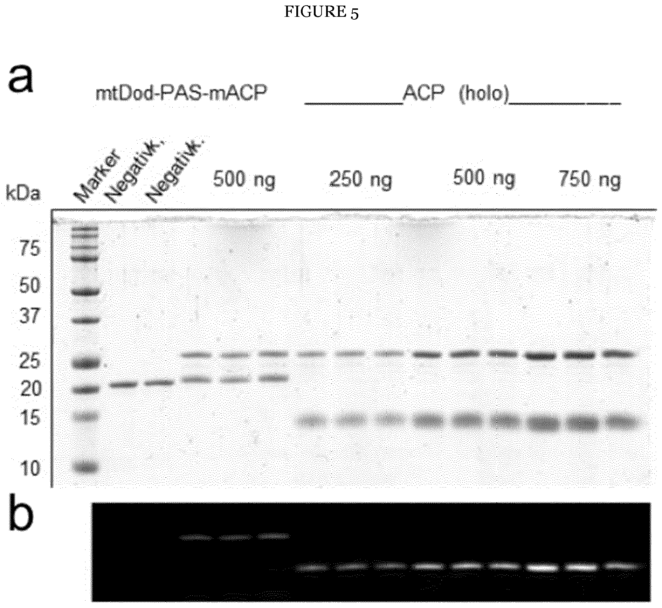

**Figure 5** shows modification of mtDod-mACP and mACP by Sfp with fluorescent CoA. a) Coomassie stained SDS PAGE gel of the reaction solution and negative controls. Upper bands (slightly above the 25 kDa ladder band) represent SFP, middle bands (slightly above the 20 kDa ladder band) mtDod-mACP and lower bands (below 15 kDa ladder band) mACP. b) Fluorescence image of the SDS PAGE gel of the reaction solutions. Only proteins modified with the ATTO dye 488 are visible as a fluorescence band. Higher bands represent mtDod-mACP and lower bands mACP.

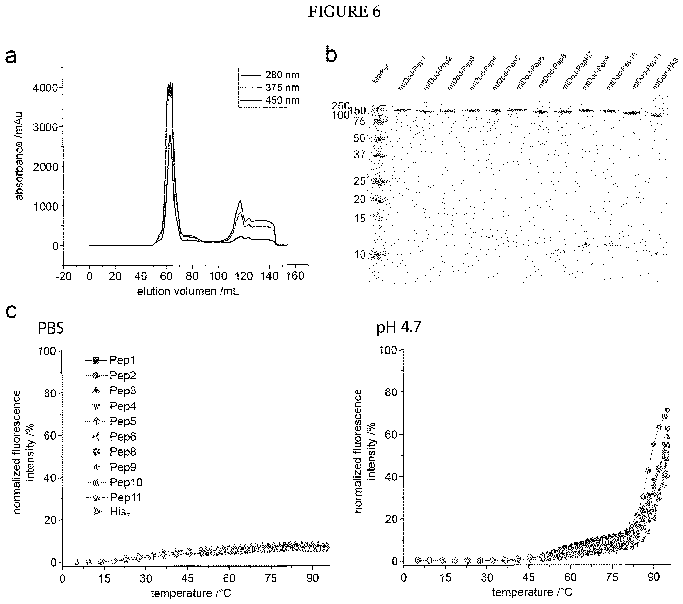

**Figure 6** shows MtDod-PAS-Pep purity and stability. a) SEC profiles of FMN:mtDod-PAS-Pepi. As only the dodecamer can bind flavins, the dodecamer peak can be identified by the absorption at 375 nm and 450 nm. In addition to the dodecamer, unbound FMN is visible at 120 mL. Except mtDod-PAS-Pep3, which formed higher oligomers in addition to the dodecamer, all constructs behaved similarly. b) SDS-PAGE gel of all purified mtDod-PAS-Pep constructs. In addition to the weak monomer band, also the dodecamer band is visible in the gel. Standard loading dye was used (30 min 95 °C; heat treatment) to obtain bands of the monomer and dodecamer. c) Thermocylic fluorescence assay of mtDod-PAS-Pep constructs.

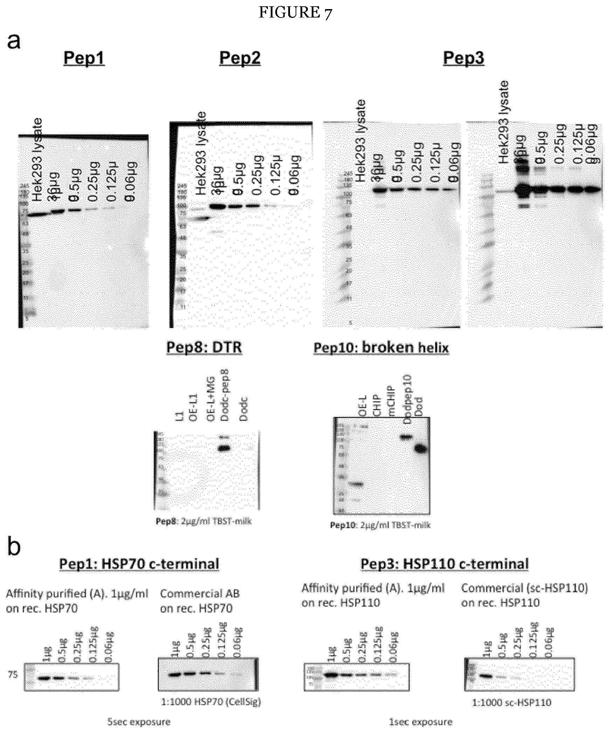

**Figure 7** shows Western Blots with selected mtDod-PAS-Pep construct antibodies. a) Overall recognition of the target protein in purified form and in lysate (L or OE-L for lysate of over expressing cells). Antibodies did not show any cross-interference, but e.g. antibodies of mtDod-PAS-Pep2 recognize HSP70 to some the degree. Antibodies derived from mtDod-PAS-Pep3 required prolonged detection times (40 s (right) compared to 13 s (left)) to detect target protein in the lysate. Antibodies derived from mtDod-PAS-Pep10 did not recognize purified target protein (CHIP), but seem to recognize something with similar mass in CHIP overexpressing cells. b) Western Blots for comparison with commercially available antibodies. Different amounts (1 µg to 60 ng) of purified target protein (here HSP70 and HSP110) were loaded and then analyzed by mtDod-PAS-Pep derived antibodies (1 µg/mL) and commercial available antibodies (manufacturer's recommended concentration).

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The following examples have been performed using dodecin from *Mycobacterium tuberculosis* (mtDod), but the person of skill will be able to readily apply and thus to transfer the teachings as herein to the inventive use of any other suitable dodecin protein.

### Example 1:

To evaluate the suitability of dodecin from *Mycobacterium tuberculosis* (mtDod) as a carrier protein, several mtDod constructs were designed and purified. All constructs were expressed in *E. coli* BL21 under the control of the lac promoter. Cells were grown in terrific broth (TB) medium to an OD of about 0.6-0.8 at 37 °C before induction with isopropyl-β-D-thiogalactopyranosid (IPTG; 0.5 mM final concentration), and expression was performed overnight at 20 °C. Cells were lysed by French press, and the cell debris was removed by centrifugation. Most mtDod constructs were produced as soluble proteins, but some constructs were received in inclusion bodies.

For soluble mtDod constructs, a heat denaturation step at about 75 °C was performed in order to remove most cytosolic *E. coli* proteins. MtDod itself is stable to temperatures above 95 °C under standard buffer conditions (pH ∼ 7.5 and ionic strength >100 mM, e.g., PBS) and the thermal stability can be further increased by native flavin mononucleotide (FMN) ligand added in excess [4, 5]. Depending on the stability of the fold of the cargo fused to mtDod, lower temperatures may be necessary during the heat denaturation step. For example, mtDod-mACP precipitates at about 55-60 °C. In this case, heat denaturation was conducted at about 55 °C. Lower temperatures during the heat denaturation step can lower the protein purities as some *E*. *coli* proteins stay in solution. In this case, purification by affinity chromatography, in order to circumvent heat treatment, may be a more suitable method.

After the removal of heat denatured and precipitated proteins by centrifugation, mtDod was generally further purified by two DMSO precipitations (50% final concentration). Here, mtDod precipitates, but can easily be dissolved in buffer again. Finally, size-exclusion chromatography (SEC) was performed to select for dodecameric fractions, identified in the chromatographic profiles by the bound flavin (absorption at 375 nm and 450 nm).

The two purification strategies; i.e., heat denaturation and affinity chromatography, were compared using the example of the construct mtDod-msfGFP-H8. GFP is a suited cargo for this test, because the high thermal stability of GFP allows heat denaturing at temperatures as used for mtDod (WT) [6]. The dodecameric structure of dodecin constructs causes a high density of affinity tags exposed at the surface allowing vigorously washing steps without severe protein loss during Ni-chelating affinity chromatography. MtDod-msfGFP-H8 was washed with 2 column volumes of a 200 mM imidazole containing wash buffer, while elution was performed at 400 mM imidazole.

Constructs that express in inclusion bodies can be refolded by dialysis as previously described in Bourdeaux *et al.* [4], with the optimal conditions depending on the fused cargo. All inclusion bodies were first washed and then dissolved by denaturation using 6 M guanidinium chloride. MtDod was refolded without further purification at different conditions ranging from pH 5.0 [4] to pH 8.5. In the context of the present examples for all constructs obtained as inclusion bodies, refolding was possible although the soluble proteins suffering from aggregation, particularly during protein concentration and filtration. For both SpyCatcher mtDod constructs a glycerol containing buffer was used.

Table 1 shows MtDod constructs that were used for expression studies. Most mtDod constructs expressed in soluble form, but some constructs formed yellowish inclusion bodies indicating partly correct folded dodecin. MtDod constructs are divided into two groups: mtDod-peptides (constructs with only short peptides fused to mtDod) and mtDod-proteins (constructs with whole proteinaceous domains or proteins fused to mtDod).

| Construct | Linker system | Molar mass /Da | Expression state |
|---|---|---|---|
| ***mt*Dod-peptides** | | | |
| *mt*Dod(WT) | - | 7497.41 | soluble |
| *mt*Dod-GSG-Lys | GSG | 8411.37 | soluble |
| *mt*Dod-PAS-Met | PAS | 8875.93 | soluble |
| *mt*Dod-SpyT | PASG | 10457-72 | soluble |
| SpyT-*mt*Dod | GPAS | 10215.45 | soluble |
| *mt*Dod-PAS2-SpyT | PAS2G | 11446.78 | soluble |
| SpyT-PAS2-*mt*Dod | GPAS2 | 11204.52 | soluble |
| SpyT-*mt*Dod-SnpT | GPAS / PASG | 13141.70 | soluble |

| ***mt*Dod-proteins** | | | |
|---|---|---|---|
| *mt*Dod-mACP | PAS | 19333.60 | soluble |
| *mt*Dod-msfGFP-H8 | PAS | 36387.68 | soluble |
| *mt*Dod-SpyC-H8* | PAS | 22412.60 | inclusion body |
| H8-SpyC-*mt*Dod | PAS | 22072.19 | inclusion body |
| *mt*Dod-SZ1 | PAS | 14231.99 | inclusion body |
| SZ3-*mt*Dod | PAS | 13395.95 | inclusion body |
| *m*tDod-*se*ACP** | PAS | 19966.22 | inclusion body |

| Linker systems | | | |
|---|---|---|---|
| GSG: | GGGGSGGGG (SEQ ID NO: 1) | PAS: | SPAAPAPASPAS (SEQ ID NO: 4) |
| PASG: | SPAAPAPASPASGGSG (SEQ ID NO: 2) | GPAS: | GGSGSPAAPAPASPAS (SEQ ID NO: 5) |
| PAS2G: | SPAAPAPASPASPAPSAPAASPAA GGSG (SEQ ID NO: 3) | GPAS2: | GGSGSPAAPAPASPASPAPSAPA ASPAA (SEQ ID NO: 6) |

| | | | |
|---|---|---|---|
| * *Mt*Dod-SpyC seems to be soluble in cellular environment, but formed yellow aggregates after cell lysis. ** *Mt*Dod-*se*ACP was not purified after refolding, because severe aggregation was observed during this step. | | | |

All constructs presented in Table 1, except mtDod-seACP, were obtained in good purity, as shown in Figure 2.

The solubility and aggregation problems observed for some constructs may possibly be solved with formation of mtDod-heterododecamers, which allows reducing the density of attached entities on the surface. It has been demonstrated that such heterododecamers can be obtained with mtDod *in vitro* and *in vivo.* In a proof of concept approach, mtDod (WT), mtDod-His and mtDod-Strep were successfully assembled to heterododecamers. For *in vitro* heterododecamer formation, the mtDod (WT) and mtDod-Strep were jointly refolded, while for the formation of heterododecamers *in vivo,* several combinations of the three mtDod constructs were coexpressed. The high stability of the dodecamer allowed observing heterododecamers by SDS PAGE, which, owing to the differently sized mtDod constructs, made all subspecies visible that reflect the different compositions.

### Example 2

A key feature of carrier proteins is a high stability that allows easy and prolonged storage. The high stability further enables the use of a wide range of conditions to conjugate cargos to the carrier. A cyclic thermal shift assay has been recently established, a termed thermocyclic fluorescence assay, to determine the stability of dodecins. This assay was applied to analyze the stability of mtDod constructs, and is based on the fluorescence quenching that is observed when flavins are bound to dodecin. In each binding pocket of the dodecamer, the two isoalloxazine ring systems of two bound flavins are embedded between symmetry-related tryptophans.[3,4,7] Since dodecins can only bind flavins in the dodecameric state, the fluorescence intensity of flavins can be used to estimate the amount of dodecameric mtDod in solution. In contrast to standard melting experiments, in which the temperature is continuously increased, the thermocyclic fluorescence assay runs cyclic temperature profiles that contain a heating phase (temperature increased per cycle) and a cooling phase (for all cycles 5 °C). At the heating phase, FMN is released from the binding pocket and the fluorescence intensity increases. After the heating phase, the sample is cooled down, and FMN can rebind to the dodecamer (cooling phase) restoring initial low fluorescence values. As soon as the dodecamer denatures during heating and refolding is prevented in the cooling phase, the fluorescence intensity remains at elevated levels. By plotting the fluorescence intensity of the cooling phase against the heating phase temperature, the thermal stability of the dodecamer of the mtDod constructs can be observed.

The thermocyclic fluorescence assay does only monitor the dodecameric stability, which, however, may be influenced by the attached cargo. As in PBS, all constructs, except mtDod-SZ1 and mtDod-SpyC, proved to be stable throughout the entire temperature range. The slightly destabilizing conditions of pH 4.2 were identified as suited to work out the impact of the cargo on the integrity of the mtDod dodecameric scaffold (see Fig. 3). Under this condition, mtDod-peptides denatured at 75-80 °C similar to mtDod wild type. The determination of the thermal stability of mtDod-proteins is more difficult, because the assay selectively addresses the mtDod dodecameric stability, but not the stability of the fused folds. Denaturation of the fused cargo may affect the FMN binding, although the dodecamer is correctly assembled, explaining the different curve shapes observed for some constructs (see Fig. 3). In screening temperatures for the heat denaturation of mtDod-mACP, for example the formation of yellow agglomerates above 55-60 °C is observed, indicating intact dodecamer that is capable of FMN binding. The high dodecameric stability of mtDod is also observed in SDS PAGE using the standard loading dye (2.5% SDS, pH 6.8). After staining, the monomer band became visible in the gel, but a dodecameric fraction remained as indicated by the high molecular weight band. In accordance to the lower stability at pH 4.2, observed in the thermocyclic fluorescence assay, a two component acidic loading dye was applied in SDS PAGE (3.3% SDS, pH 4.2 at heat treatment step, afterwards 2.5% SDS; pH 6.8) capable of fully denaturing the dodecamer. Figure 2 shows the results of the SDS PAGE.

Depending on the use of the carrier matrix, storage conditions may be relevant, too. It was observed that most mtDod constructs can be frozen and thawed several times without significant aggregation. A glycerol containing buffer was used for the SpyCatcher constructs, and mtDod-PAS-msfGFP showed minor formation of green fluorescent aggregates.

### Example 3

The accessibility and functionality of folds and peptides fused to mtDod were tested by the reactivity of the SpyTag/-Catcher pair (also SnoopTag/-Catcher pair). By equipping proteins/peptides with a short peptide (Tag) and a small protein fold (Catcher), that form a covalent bond upon interaction, proteins and/or peptides can be stably fused [8]. Applications range from attaching proteins from pathogens to scaffolds like virus like particles and IMX313 (heptamer forming coiled coils) for immunizations [9,10] to attaching enzymes to a scaffold in order to create enzyme hubs of increased catalytic efficiency [11].

For the SpyTag/-Catcher and SnoopTag/-Catcher reaction with the tagged mtDod constructs, seACP-SpyCatcher and mClover3-SnoopCatcher were prepared as cargo. For the inverse reaction of mtDod-SpyCatcher constructs, SpyTag-seACP was used as a cargo. For all reactions, two molar equivalents of cargo were used to promote the saturation of scaffold with cargo. The reactions were incubated for 20 h at 22 °C and analyzed by SDS PAGE. The results are shown in Figure 4.

For all combinations of mtDod scaffold and cargo, the expected product band (or bands) of mtDod and the specific cargo (or two cargos) were observed in SDS PAGE. While for mtDod Spy-/SnoopTag constructs, no significant amount of unreacted scaffold proteins was observed, for the counterpart mtDod SpyCatcher constructs, bands of unreacted scaffold monomer were visible. It was shown that mtDod Spy-/SnoopTag constructs are lower in molecular mass as compared to the mtDod SpyCatcher constructs, and traces of unreacted scaffold protein may be less visible in SDS PAGE.

The data shows that a high degree of saturation was achieved, indicating that SpyTag/- Catcher or SnoopTag/-Catcher are accessible at the mtDod dodecamer scaffold and allowing the loading of cargo with high efficiency. For the double-tagged constructs SpyT-mtDod-SnpT or SnpT-mtDod-SpyT, heterovalently loaded with seACP-SpyCatcher and mClover3-SnoopCatcher, SDS PAGE reveals bands of single charged mtDod monomers. Since the surface density is increased by the double-tagging of mtDod, in this case the crowding of the scaffold surface seems to sterically limit the degree of conjugation with both cargos.

### Example 4

A standard application of protein carriers lies in the production of antibodies against peptides or proteins [12]. Following the general procedure, the peptide or the protein of interest is linked to the carrier, usually BSA, keyhole limpet hemocyanin (KLH) or ovalbumin (OVA), by chemical ligation [12,13]. While the method is in general successful and widely used for antibody production, there can be problems related to the conjugation of antigen and carrier, like low stability of the conjugate or altered antigenic properties of the peptide.[14] The dodecameric structure with the exposed termini allows mtDod to be charged with 12 or 24 peptides/proteins on its surface by simply fusing the peptide/protein encoding sequence to the mtDod gene. As demonstrated with an initial set of constructs, as schematically shown in Figure 1, the mtDod scaffold can readily be expressed as soluble protein and easily purified by heat denaturation. In some cases, *in vitro* refolding of the dodecamer was required, because the constructs formed inclusion bodies. In order to evaluate the suitability of mtDod for antibody production, 11 fusion constructs, comprised of an N-terminal peptide, a linker PAS linker sequence and mtDod, were produced in *E. coli* and used for antibody production. The following table 2 shows the mtDod constructs used for the antibody production.

**Table 2: MtDod constructs for antibody production. After purification by the heat treatment protocol constructs were verified by LCMS.**

| *mt*Dod constructs | Peptide sequence | calculated mass without start-Met/Da | measured mass by LCMS (+1H⁺)/Da |
|---|---|---|---|
| *mt*Dod-PAS-Pep1 | PKGGSGSGPTIEEVD (SEQ ID NO: 7) | 10155 | 10156.7 |
| *mt*Dod-PAS-Pep2 | PLEGDDDTSRMEEVD (SEQ ID NO: 8) | 10434 | 10435.0 |
| *mt*Dod-PAS-Pep3 | ECYPNEKNSVNMDLD (SEQ ID NO: 9) | 10497 | 10803.4* |
| *mt*Dod-PAS-Pep4 | VPSDSDKKLPEMDID (SEQ ID NO: 10) | 10415 | 10416.1 |
| *mt*Dod-PAS-Pep5 | DSSQHTKSSGEMEVD (SEQ ID NO: 11) | 10363 | 10363.9 |
| *mt*Dod-PAS-Pep6 | EQSTGQKRPLKNDEL (SEQ ID NO: 12) | 10469 | 10470.2 |
| *mt*Dod-PAS-Pep7** | ALMVYRCAPPRSSQF (SEQ ID NO: 13) | 10453 | |
| *mt*Dod-PAS-Pep8 | LVTGESLEQLRRGLA (SEQ ID NO: 14) | 10368 | 10369.1 |
| *mt*Dod-PAS-Pep9 | MKGKEEKEGGARLGA (SEQ ID NO: 15) | 10287 | 10288.0 |
| *mt*Dod-PAS-Pep10 | EERRIHQESE (SEQ ID NO: 16) | 10038 | 10039.6 |
| *mt*Dod-PAS-Pep11 | NHEGDEDDSH (SEQ ID NO: 17) | 9880 | 9881.3 |
| *mt*Dod-PAS-H7 | HHHHHHH (SEQ ID NO: 18) | 9704 | 9705.2 |

| | | | |
|---|---|---|---|
| *Difference of mass is about 305 Da and could be caused by S-glutathionylation (15, 16). No mass for the unmodified *mt*Dod-PAS-Pep3 was observed. **mtDod-PAS-Pep7 formed inclusion bodies and was not further purified. | | | |

For cloning, the Peptide-encoding sequence was provided on primer sequences and introduced in a single step by ligation free cloning methods. Recombinant expressions and purifications followed the established protocols. All constructs were received as soluble proteins, except mtDod-PAS-Pep7 that formed inclusion bodies. The yellow color of the inclusion bodies indicated assembled dodecamer and aggregation most likely induced by disulfide-bridges formation by the cysteine in the Pep7 sequence. All constructs, except mtDod-PAS-Pep7, were further purified by two cycles of DMSO-induced precipitations. FMN was added before all constructs were finally purified by SEC to remove unbound FMN and remaining DMSO as well as to select for dodecameric species. For example, the SEC profiles of FMN:mtDod-PAS-Pepi is shown in Figure 6a. FMN-saturated mtDod constructs (FMN:mtDod constructs) can be determined in concentration by absorbance at 450 nm and are susceptible to stability measurements by the thermocyclic fluorescence assay. All constructs were received as dodecamers as indicated by SEC. MtDod-PAS-Pep3 shows in addition to the dodecamer species higher oligomeric states in SEC. The dodecamer containing fractions were pooled and the purity was controlled by SDS-PAGE. Figure 6b shows the thermocyclic fluorescence assay of mtDod-PAS-Pep constructs. Molecular masses of the constructs were verified with LCMS. The above mentioned Table 2 shows the results.

The yield of each mtDod construct was between 150-500 mg per liter *E. coli* expression culture (values extrapolated as only a fraction was purified, absolute yields 20-50 mg). With 20 mg, MtDod-PAS-Pep3 was received in the lowest yield, which may originate from aggregation of dodecamers, induced by disulfide bridges formed by a cysteine in the Pep3 sequence. In general, the constructs mtDod-PAS-Pep3 and mtDod-PAS-Pep7 indicate that cysteine containing peptides can cause problems when processed via the described purification strategy, owing to the oxidative conditions imposed by FMN. Accordingly, a changed protocol that avoids working with FMN in excess and/or that includes reducing agents should make those constructs accessible. The thermocyclic fluorescence assay, showed the high thermal stability of all mtDod-PAS-Pep constructs, similar as the wild type [4]. The results are shown in Figure 6c.

Endotoxin concentrations, measured in endotoxin units (EU) via a Limulus amebocyte lysate (LAL) test, were determined to avoid an endotoxin shock in immunizations. Dod-PAS-Pep3 and Dod-PAS-Pep6 contained the highest amount of endotoxin with 73 EU/mg and 55 EU/mg, respectively; all other samples showed values less than 30 U/mg (average of all constructs 30 ± 23 U/mg). Since less than 0.1 mg protein was used per injection, none of the samples were in the critical range to cause an endotoxin shock (The non-pyrogenic amount of endotoxin is less than 5 EU/kg. A rabbit used for immunization weighs on average 5 kg, so the amount per injection should be less than 25 EU (=0.25 ng LPS/ml)) [15, 16]. Antibodies were produced in rabbits, and immunizations conducted with 5 boosts during 63 days and using adjuvants MF59/AddaVax or Montanide ISA 51. The antibodies were purified by affinity chromatography with the respective Dod-PAS-Pep construct immobilized on the column matrix. For the 10 Dod-PAS-Pep constructs subjected to immunizations, purified antibodies were obtained. 6 of the 10 antibodies recognized the peptide containing target protein either as heterologously expressed protein or as protein part of HEK293T lysate. Figure 7a shows that the antibodies overall showed no systematic off/background targeting in the used lysates, indicating that mtDod-PAS is a suitable matrix in this respect. The 4 remaining antibodies (constructs) did not recognize the peptide containing target protein and only recognize the mtDod-PAS matrix and the respective mtDod-PAS construct. In Western Blotting, the target recognizing antibodies performed comparably to available commercial antibodies. This is shown in Figure 7b. For all target-recognizing antibodies, the recognition limit/range of purified recombinant protein was analyzed and compared with commercially available antibodies.

Table 3: Recognition strength of mtDod-PAS-Pep construct antibodies. The lowest amount of loaded purified recombinant protein (in ng) that could be clearly labeled with the respective antibody was used as detection limit. MtDod-PAS-Pep construct derived antibodies were used at a final concentration of 1 µg/mL and commercial antibodies at the recommended dilution. For comparison the exposure time for each antibody pair (mtDod-PAS-Pep construct derived and commercial) was the same, while it was varied for the different pairs.

| antibody derived form *mt*Dod construct | target site of the antibody | lowest amount of recombinant protein clearly labelled /ng | |
|---|---|---|---|
| | | derived antibody | commercial antibody |
| *mt*Dod-PAS-Pepi | HSP70 c-terminal | 125-250 | 60-125 |
| *mt*Dod-PAS-Pep2 | HSP90 c-terminal | 125-250 | - |
| *mt*Dod-PAS-Pep3 | HSP110 c-terminal | 60 | 250-500 |
| *mt*Dod-PAS-Pep4 | H2 c-terminal | 60-125 | 125-250 |
| *mt*Dod-PAS-Pep5 | H3 c-terminal | 500* | - |
| *mt*Dod-PAS-Pep6 | H4 c-terminal | - | - |
| *mt*Dod-PAS-Pep8 | DTR | - | - |
| *mt*Dod-PAS-Pep9 | CHIP N-term | 125-250 | 60 |
| *mt*Dod-PAS-Pep10 | CHIP broken helix | -** | - |
| *mt*Dod-PAS-Pep11 | CHIP tip of helix | 60-125 | 60 |

| | | | |
|---|---|---|---|
| * *mt*Dod-PAS-Pep5 derived antibodies showed only a very weak signal for 1 µg and 500 µg of recombinant protein with no intensity difference. While something is recognized the antibody preparation was counted as not target recognizing. ** *mt*Dod-PAS-Pep10 derived antibodies didn't recognize purified CHIP, but seem to recognize a protein in CHIP overexpressing cells, no detection range was determined. | | | |

The mtDod-PAS-Pep derived antibodies show that mtDod-PAS is suitable as a carrier system for the production of peptide specific antibodies. Key benefits of mtDod-PAS as a carrier are the easy cloning, uncomplicated production/purification and the high yields. Problems in the purification of constructs that occur from the oxidative conditions caused by the high FMN concentration; i.e., promoting disulfide formation between exposed cysteines of the peptide tags, may be avoided when working under reducing conditions.

### REFERENCES

1. B. Bieger, L.-O. Essen, D. Oesterhelt, Structure 2003, 11, 375-385.
2. M. Grininger, H. Staudt, P. Johansson, J. Wachtveitl, D. Oesterhelt, J. Biol. Chem. 2009, 284,13068-13076.
3. B. Meissner, E. Schleicher, S. Weber, L. O. Essen, J. Biol. Chem. 2007, 282, 33142-33154.
4. F. Bourdeaux, C. A. Hammer, S. Vogt, F. Schweighöfer, G. Nöll, J. Wachtveitl, M. Grininger, ACS Infect. Dis. 2018, 4, 1082-1092.
5. F. Liu, J. Xiong, S. Kumar, C. Yang, S. Ge, S. Li, N. Xia, K. Swaminathan, J. Struct. Biol. 2011, 175, 31-38.
6. M. Ishii, J. S. Kunimura, H. T. Jeng, T. C. V. Penna, O. Cholewa, in Appl. Biochem. Biotecnol., Springer, 2007, pp. 555-571.
7. M. Grininger, K. Zeth, D. Oesterhelt, J. Mol. Biol. 2006, 357, 842-857.
8. L. Li, J. O. Fierer, T. A. Rapoport, M. Howarth, J. Mol. Biol. 2014, 426, 309-317.
9. K. D. Brune, D. B. Leneghan, I. J. Brian, A. S. Ishizuka, M. F. Bachmann, S. J. Draper, S. Biswas, M. Howarth, Sci. Rep. 2016, 6, DOI 10.1038/srep19234.
10. K. D. Brune, C. M. Buldun, Y. Li, I. J. Taylor, F. Brod, S. Biswas, M. Howarth, Bioconjug. Chem. 2017, 28, 1544-1551.
11. Jia Lili, Minamihata Kosuke, Ichinose Hirofumi, Tsumoto Kouhei, Kamiya Noriho, Biotechnol. J. 2017, 12, 1700195.
12. N. H. Trier, P. R. Hansen, G. Houen, Methods 2012, 56, 136-144.
13. J. M. Peeters, T. G. Hazendonk, E. C. Beuvery, G. I. Tesser, J. Immunol. Methods 1989, 120, 133-143.
14. J. P. Briand, S. Muller, M. H. V. Van Regenmortel, J. Immunol. Methods 1985, 78, 59-69.
15. R. E. Wachtel, K. Tsuji, Appl. Environ. Microbiol. 1977, 33, 1265-1269.
16. F. C. Pearson, M. E. Weary, H. E. Sargent, T. J. Novitsky, H. Lin, G. Lindsay, R. N. Berzofsky, A. L. Lane, J. D. Wilson, J. F. Cooper, Appl. Environ. Microbiol. 1985, 50, 91-93.

## Claims

1. A conjugate comprising at least one dodecin protein unit conjugated with at least one hapten and/or at least one immunogenic and/or at least one enzymatically active moiety.

2. The conjugate according to claim 1, wherein said at least one hapten and/or at least one immunogenic and/or at least one enzymatically active moiety is complexed with and/or genetically fused to said dodecin protein unit.

3. The conjugate according to claim 1 or 2, wherein said dodecin protein is a dodecamer of twelve units of said dodecin protein, wherein said dodecamer comprises at least one conjugated dodecin protein unit conjugated with said at least one hapten and/or at least one immunogenic and/or said at least one enzymatically active moiety, wherein preferably both termini of the amino acid chain of said dodecin protein unit are located on the outer surface of said dodecamer.

4. The conjugate according to any one of claims 1 to 3, wherein said moiety is selected from an antigen, a peptide, an enzyme, a protein, a lipid, and a small molecule.

5. The conjugate according to any one of claims 1 to 4, wherein said conjugation is covalent or non-covalent via a functional group, wherein said functional group preferably is selected from a thiol group, an amino group, a carboxy group and an azide group.

6. The conjugate according to any one of claims 1 to 5, wherein said fusion is direct or indirect via a suitable linker group or sequence, for example a peptide linker.

7. The conjugate according to any one of claims 1 to 6, wherein said dodecin protein unit conjugated with said at least one hapten and/or at least one immunogenic and/or at least one enzymatically active moiety is suitably labelled.

8. The conjugate according to any one of claims 1 to 10, wherein said dodecin protein unit is derived from a bacterium or archaea selected from *Halobacterium salinarum, Halobacterium halobium, Streptomyces davaonensis, Streptomyces coelicolor, Chlorobium tepedium, Sinorhizobium meliloti, Bordetella pertussis, Bordetella bronchiseptica, Pseudomonas aeruginosa, Pseudomonas putida, Acinetobacter baumannii, Thermus thermophilus, Geobacter sulfurreducens, and* preferably from *Mycobacterium tuberculosis.*

9. A composition comprising the conjugate according to any one of claims 1 to 8 and at least one of a suitable carrier, excipient, enzyme substrate or adjuvant.

10. A nucleic acid encoding for a conjugate according to any one of claims 1 to 8, or a vector comprising said nucleic acid, in particular an expression vector expressing or overexpressing said nucleic acid.

11. A recombinant host cell, comprising the nucleic acid or the vector according to claim 10.

12. A method for producing a conjugate according to any one of claims 1 to 8, the method comprising suitably culturing a recombinant host cell comprising and expressing the nucleic acid or the vector according to claim 10, and isolating said conjugate from the cell and/or the culture medium thereof.

13. A method for producing a conjugate according to any one of claims 1 to 8, the method comprising suitably culturing a recombinant host cell comprising and expressing a nucleic acid or a vector encoding a dodecin protein unit, isolating said dodecin protein unit; and complexing an hapten and/or immunogenic and/or enzymatically active moiety via a functional group to said isolated dodecin protein unit.

14. A method for producing an antibody that specifically binds to the hapten of the conjugate according to any one of claims 1 to 8, comprises the steps of suitably immunizing a subject with the conjugate according to any one of claims 1 to 8, and isolating an antibody specifically binding to the hapten of said conjugate from the subject, wherein said subject preferably is a mammal.

15. A method for performing an enzymatic, preferably a diagnostic, assay *in vitro,* wherein said method comprises the steps of immobilizing at least one conjugate comprising an enzymatically active moiety according to any one of claims 1 to 8 on a solid carrier; adding a suitable enzyme substrate; and determining the amount of converted enzyme substrate, wherein preferably said solid carrier is selected from glass, agarose, polymers, and metal.
